## Europäisches Patentamt
## European Patent Office
## Office européen des brevets

(19)

(11) Numéro de publication: **0 106 831**
**B1**

(12) # FASCICULE DE BREVET EUROPÉEN

(45) Date de publication du fascicule du brevet:
27.12.89

(51) Int. Cl.⁴: **A 61 K 39/15,** A 61 K 39/155,
C 12 N 7/06, C 12 N 15/00

(21) Numéro de dépôt: 83870106.8

(22) Date de dépôt: 11.10.83

(54) Souches modifiées du virus de la diarrhée virale bovine, leur préparation et vaccins les contenant.

(30) Priorité: 13.10.82 US 434155

(43) Date de publication de la demande:
25.04.84 Bulletin 84/17

(45) Mention de la délivrance du brevet:
27.12.89 Bulletin 89/52

(84) Etats contractants désignés:
AT BE CH DE FR GB IT LI LU NL SE

(56) Documents cité:
FR-A-2 210 387
US-A-3 293 129
US-A-3 629 413
US-A-3 962 424

BIOLOGICAL ABSTRACTS, vol. 74, no. 3, 1982, page 1822, réf.no. 17524, R. WEIBLEN et al.: "Comparative study of vaccines in preventing respiratory disease in beef calves after weaning.", & RES COMMUN. CHEM. PATHOL PHARMACOL 35(2): 303-324. 1982.
BIOLOGICAL ABSTRACTS, vol. 71, no. 8, 1981, page 5546, réf.no. 52825, F. STECK et al.: "Immune responsiveness in cattle fatally affected by bovine virus diarrhea-muscosal disease.", & ZENTRALBL. VETERINAERMED REIHE B 27(6): 429-446, 1980

Le dossier contient des informations techniques présentées postérieurement au dépôt de la

(73) Titulaire: **SMITHKLINE BECKMAN - ANIMAL HEALTH PRODUCTS, Rue du Tilleul, 13, B-1320 GENVAL (Rixensart) (BE)**

(72) Inventeur: **Delgoffe, Jean- Claude, Rue Joseph Francis, 54, B-1301 Bierges (BE)**
Inventeur: **Lobmann, Michèle, Rue Rosier- Bois, 4, B-1331 Rosières (BE)**
Inventeur: **Zygraich, Nathan, Avenue H. Boulanger, 49, B-1180 Bruxelles (BE)**

(74) Mandataire: **Tasset, Gérard, Smithkline Biologicals s.a. Rue de l'Institut 89, B-1330 Rixensart (BE)**

(56) Documents cité: (suite)
demande et ne figurant pas dans le présent fascicule.

EP 0 106 831 B1

## Description

La présente invention concerne des souches modifiées du virus de la diarrhée virale bovine (DVB), leur préparation et les vaccins les contenant.

La diarrhée virale bovine est l'une des maladies du bétail les plus répandues et les plus importantes. Elle a d'abord été signalée aux Etats-Unis d'Amérique (OLAFSON et al., Cornell Vet. 36 : 205 - 213, 1946) lorsqu'on a observé parmi les troupeaux de bovins les premières manifestations d'un syndrome aigu, souvent fatal et comparable à la peste bovine, avec ulcérations de la muqueuse du tube digestif et diarrhée. En même temps, des cas similaires, présentant différents degrés de gravité, de chronicité et de sporadicité étaient décrits et dénommés maladie des muqueuses (RAMSEY F.K. et al., North Am. Vet. 34 : 629 - 633, 1953). Pendant un certain temps, on a pensé qu'il s'agissait d'agents infectieux différents. Par la suite, on est arrivé à la conclusion que c'était un même virus qui était responsable des deux syndromes mais, à ce moment, les deux terminologies étaient enracinées dans la littérature et le terme complexe de maladie des muqueuses était déjà passé dans le langage courant.

Sur base de la priorité, la maladie fut dénommée diarrhée virale bovine.

L'agent étiologique de DVB est un virus ARN classifié comme un pestivirus de la famille Togaviridae (ANDREWES C. et al., Viruses of the Vertebrates, 4th ed., London; Bailliere Tindall, 1978).

Les manifestations cliniques de la maladie sur le terrain varient considérablement. En général, une période d'incubation de 7 - 11 jours est suivie d'une pyrexie mono- ou di-phasique, de 39,5 - 41,5°, anorexie et diarrhée.

Parfois, la diarrhée progresse jusqu'à l'évacuation de nourriture non digérée, de mucus floconneux hémorragique et de membranes diphtériques, accompagnée de violent ténesme. Malgré une polydipsie concomitante, les animaux se déshydratent et perdent rapidement leur forme.

Bien que la DVB soit principalement associée à une infection du tractus digestif, il est apparu que la séquence des symptômes commence habituellement par un épisode respiratoire: un jetage nasal séreux ou muqueux devenant par la suite purulent et accompagné de polypnée et de toux (S.F. ROSNER, Iowa Vet. 35 : 11 - 14, 1964). De nombreux auteurs ont également signalé un jetage nasal progressant du type séreux au type mucopurulent.

Les principaux changements pathologiques associés à la maladie sont une hyperémie, une hémorragie, un oedème, une érosion et ulcération de la muqueuse digestive. On a également observé une congestion des surfaces muqueuses du tractus respiratoire ainsi qu'un oedème pulmonaire. Généralement, une forte leucopénie est également présente, particulièrement au cours des premiers stades de l'infection. On a observé que les comptages de globules glancs sanguins revenaient à la normale environ 14 jours après infection expérimentale par inoculation (SCOTT F.W. et al., Cornell Vet. 63 : 536 - 560, 1973).

Sur ces bases, MILLS J.H.L. et al. (Am. J. Vet. Res. 29 : 1367 - 1375, 1968) ont suggéré que "la transmission par la voie respiratoire est un mode possible de propagation".

On a incriminé une immunotolérance congénitale à l'infection par le virus de la DVB (VDB) comme cause potentielle de la maladie rarement mortelle et il est reconnu qu'une immunodépression induite par un traitement aux corticoïdes potentialise la virulence d'une infection artificielle avec le VDB. Les animaux immunodéprimés succombent souvent d'une virémie généralisée (RHODE, G et al., Sbl. Vet. Med. 17B : 686, 1970, et SHOPE R.E. et al., Can. J. Comp. Med. 40 : 355 - 359, 1976).

Dès que des signes cliniques évidents apparaissent dans un troupeau, l'infection est généralement bien établie chez les individus affectés ainsi que dans le troupeau. On ne connaît pas de mesure thérapeutiques spécifiques pour traiter les cas cliniques. S'il s'agit de cas sévères, ils succombent généralement. S'il s'agit de cas bénins, on peut accélérer le rétablissement par l'administration d'antibiotiques pour contrôler une infection secondaire.

Dans les conditions ordinaires d'exploitation, il s'est avéré inadéquat de maintenir un troupeau fermé pour prévenir une infection par VDB. C'est pourquoi les méthodes préventives sont largement basées sur des vaccins.

Les vaccins inactivés ont fait l'objet d'une recherche considérable (McCLURKIN A.W. et al., Pro. US Anim. Health Assoc. 79 : 114 - 123, 1975). Jusqu'ici, ceux-ci ne sont pas disponibles commercialement. Leur utilisation pratique est compromise par le manque de technologie adéquate pour la production de larges quantités de virus nécessaires à fournir d'une manière économiquement acceptable les masses antigéniques requises.

On connaît quelques vaccins VDB vivants commerciaux. Comme l'indiquent R.M. PHILLIPS et al. (Am. J. Vet. Res. 36 : 135 - 140, 1975), "le premier développement de vaccin pour le contrôle de la DVB est basé sur l'atténuation de l'agent viral par des passages en série sur des cultures de cellules rénales de lapin ou de bovin. Ces vaccins se sont révélés efficaces dans la prévention d'infections virales par VDB chez les bovidés mais on a fait état de complications survenant après vaccination. Dans le même article, les auteurs décrivent également le développement d'un vaccin viral à base de VDB vivant atténué produit sur une lignée cellulaire porcine.

Néanmoins, les vaccins contre la DVB actuellement disponibles n'évitent pas les autres risques inhérents aux vaccins précédemment connus (TERPSTRA, C. EIKELENBOOM, J.L. and GLAS, C. Proceedings of the WIIth World Congress on diseases of cattle, Vol. 1 : 177, 1982).

Pour essayer d'expliquer les sévères complications postvaccinales qui se présentent à des fréquences variables sur le terrain, on a avancé plusieurs raisons plausibles, différentes de la virulence de la souche vaccinale elle-même; néanmoins, leur origine est difficile à établir et les vaccins ont été sévèrement mis en cause.

La présente invention évite les risques des vaccins vivants contre la DVB connus jusqu'ici, en mettant à disposition des souches qui sont des souches mutantes thermosensibles (ts) du VDB, c'est-à-dire des souches qui montrent une faculté de replication considérablement limitée à la température du corps de l'animal et qui sont néanmoins capables d'induire une immunité chez les bovins sans entraîner d'effets secondaires sérieux.

Les souches modifiées de la présente invention sont préparées par mutagénèse de souches de VDB avec un agent mutagène chimique tel qu'un traitement à l'acide nitreux de VDB isolé d'un animal infecté, p. ex. une souche de VDB sauvage typique, isolée et déposée dans la "Collection Nationale de Cultures de Microorganismes" de l'Institut Pasteur à Paris le 2 juillet 1982 sous le numéro d'ordre I - 198, en accord avec les dispositions de la Convention sur le Brevet Européen.

La mutagénèse par agents chimiques est une technique connue des spécialistes et, parmi les agents chimiques connus pour induire une mutagénèse et pouvant être employés dans le procédé de cette invention, on peut citer par exemple, à côté de l'acide nitrique cité plus haut, la N-méthyl-N-nitroso-N-nitro guanidine, le sulfonate de méthylméthane, le sulfonate d'éthylméthane, le 5-bromo uracyle, la 2-amino purine, l'hydroxylamine et des colorants acridiniques comme la 5-amino acridine et la proflavine. Les brevets N° 3 907 986 et 3 962 424 des Etats-Unis d'Amérique décrivent plus particulièrement la préparation de virus mutants thermosensibles par traitement à l'acide nitrique et l'utilisation desdits mutants dans la préparation de vaccins viraux vivants utiles à la prévention de maladies respiratoires chez les bovins. Néanmoins, avant cette invention, on ne connaissait pas de mutants thermosensibles du VDB.

Selon la présente invention, pour préparer une souche vaccinale au départ d'une souche de VDB sauvage typique, la souche est amenée à réagir avec l'agent mutagène - p. ex. de l'acide nitrique - dans des conditions opératoires qui réduisent le titre initial en virus de 2 à 3 $\log_{10}$ et, en cultivant respectivement à 35°C et à 39,5°C les mutants obtenus, on isole un mutant qui montre à 39,5°C (qui est la température des organes internes des bovins) une capacité de croissance qui est environ 3 $\log_{10}$ moindre que sa capacité de croissance à 35°C. Le virus mutant thermosensible est isolé par clonage dans n'importe quelle culture de tissu connue dans la pratique de l'art pour supporter la multiplication du VDB (par exemple, la lignée de cellules rénales bovines qui est déposée sans aucune restriction d'accès à l'American Type Culture Collection sous le numéro d'ordre ATCC CC 144 ou un tapis monocellulaire de cellules de cornets de bovin - tel que décrit par McCLURKIN A.W. et al., dans Arch. Gesam. Virusforsch. 45 : 285 - 289, 1974) à une dilution appropriée pour permettre la sélection de clones par passage en dilution limite.

Plus particulièrement, une souche typique sauvage isolée d'un animal présentant des symptômes typiques de la diarrhée virale bovine - par exemple, la souche de virus C.N.C.M. I - 198 - est multipliée dans une culture tissulaire permettent le développement du virus de la diarrhée virale bovine - p. ex. une lignée cellulaire comme une lignée de tissu rénal bovin ou une lignée de cellules de cornets - éventuellement après adaptaon de la souche à ladite culture de tissu et on fait réagir une suspension dudit VDB avec un agent mutagène, par exemple une solution aqueuse tamponnée d'acide nitreux (p. ex. de l'acide nitreux dans un tampon acétique, les concentrations en acide nitreux et en ion acétate dans le milieu respectivement 4N et N), à température ordinaire pendant une à 15 minutes, p. ex. pendant 6 (± 0,1) minutes, à un pH compris entre 5 et 6 (p. ex. à pH 5,7 (± 0,1)) après quoi, la réaction est arrêtée, p. ex. par ajustement du pH à 7,5 (± 0,5) par addition goutte à goutte d'hydroxyde de sodium en solution aqueuse normal. Ensuite, la solution est, de préférence, dialysée et on fait croître des aliquotes diluées dans une culture de tissu, comme indiqué plus haut. Les cultures positives sont alors multipliées et titrées comparativement à 35°C et 39,5°C. On choisit ensuite une culture dont les titres aux deux températures montrent une différence d'environ 3 $\log_{10}$ DICT$_{50}$ (doses infectieuses en culture de tissu). Un tel mutant thermosensible est alors éventuellement multiplié dans une culture tissulaire permettant le développement du virus, comme indiqué plus haut, et cloné par au moins un passage en dilution limite, de manière à isoler le mutant thermosensible, p. ex. la souche virale C.N.C.M. I - 199.

Un essai en laboratoire, effectué sur des animaux immunodéprimés avec un vaccin contenant la souche I - 199 a apporté une solide évidence de ce que le vaccin suivant la présente invention présente une amélioration significative par rapport aux vaccins existants antérieurement.

Les symptômes observés avec la souche C.N.C.M. I - 199 se situent en effet à un niveau remarquablement bas, contrastant avec le tableau clinique sévère observé chez des animaux immunodéprimés et infectés avec des souches soit virulentes, soit "atténuées" de VDB. Des résultats d'études avec des souches virulentes ont été publiées par SHOPE R.E. et al., dans Can. J.Comp. Med. 40 : 355 - 359, 1976 et des résultats d'études avec une souche atténuée ont été publiés par BITTLE J.S. et HOUSE J.A., J.A.V.M.A. 163 : 878 - 879, 1973 dans ce modèle animal. Dans ce dernier cas, BITTLE a traité deux veaux à la dexaméthasone pendant 11 jours et a noté que les animaux restaient normaux jusqu'au moment de la vaccination avec un "virus atténué (C24V)" et montraient alors des signes typiques de la maladie des muqueuses, accompagnés de fièvre et de leucopénie dès que le traitement immunodépressif était arrêté. Un animal est mort 24 jours après vaccination alors que l'autre a fini par se rétablir.

Dans l'essai où on a utilisé, chez des veaux immunodéprimés, la souche C.N.C.M. I - 199 de cette invention, on n'a constaté aucun signe clinique typique de la maladie des muqueuses, à l'exception d'une diarrhée d'intensité variable qui disparut sans incidents. On n'a cependant pas reisolé de VDB des animaux vaccinés en dépit d'essais journaliers de réisolement de virus pendant la période où les problèmes digestifs étaient présents. Ces observations cliniques n'étaient donc pas associées à la présence de virus vaccinal dans les matières fécales ou dans les sécrétions oculonasales des vaccinés. Les symptômes observés n'étaient pas non plus asso-

EP 0 106 831 B1

ciés à une virémie. Il convient également de noter qu'on n'a pas observé d'apparition de virus vaccinal génétiquement altéré. De plus, contrairement aux vaccins précédemment connus dont l'ingrédient actif est un mutant thermosensible administré à un site relativement froid de l'organisme, les vaccins de la présente invention ne sont pas infectants lorsqu'ils sont ainsi administrés et ils doivent nécessairement être administrés par voie parentérale classique, c'est-à-dire soit par voie sous-cutanée, soit dans un muscle de l'animal et à condition que ladite administration parentérale soit suivie d'une injection de rappel (booster), par exemple quelques semaines plus tard.

La dose unitaire d'un vaccin suivant l'invention contient, de préférence, au moins $10^{3,5}$ DICT$_{50}$ et, mieux encore, au moins $10^4$ DICT$_{50}$ de virus modifié. Des essais en laboratoire, effectués avec un vaccin combiné contenant du VDB modifié obtenu suivant le procédé de la présente invention et un autre virus vaccinal vivant respiratoire pour bovins et administrable par voie parentérale (en l'occurrence, du virus vaccinal contre les infections dues au virus respiratoire syncytial bovin ou RSB) ont montré que le virus VDB modifié n'interfère pas - c'est-à-dire n'affecte pas l'immunogénicité de l'autre virus vaccinal et, en conséquence, un autre aspect de l'invention concerne les vaccins viraux vivants combinés pour bovins qui comprennent un mutant VDB comme indiqué ci-avant et un autre virus vaccinal pour bovins et administrable par voie parentérale.

L'invention est illustrée par les exemples suivants qui ne doivent pas être compris comme une limite de l'invention.

**Exemple 1**

**Mutagénèse et sélection d'un mutant**

Une souche de virus de la diarrhée bovine prélevée sur la muqueuse nasale d'un veau présentant des symptômes typiques de la maladie a été adaptée à la culture de cellules testiculaires de veau par 10 passages dans une telle culture de tissu et déposée le 20 juillet 1982 en accord avec les dispositions de la Convention sur le Brevet Européen (CBE) dans la Collection Nationale de Cultures de Microorganismes (C.N.C.M.) de l'Institut Pasteur à Paris et a reçu le numéro d'ordre I - 198.

Le virus a ensuite été multiplié par 6 passages sur une lignée de cellules rénales de bovin reprise ici sous l'appellation NLBK$_4$, en utilisant un milieu constitué d'un mélange 50/50 (v/v) de milieu de base et minimal de Eagle additionné de 0,25 % (poids/volume) d'hydrolysat de lactalbumine bovine.

On a mélangé un volume de 1,25 ml d'une suspension de virus provenant du 16ème passage et contenant $10^{4,0}$ DICT$_{50}$/0,1 ml avec 1,25 ml d'une solution tampon acide acétique/acétate et sodium molaire et 1,25 ml d'une solution tampon acide acétique/acétate de sodium molaire et 1,25 ml d'une solution aqueuse 4 molaire de nitrite de sodium. Le pH final du mélange était 5,7. Le mélange a réagi pendant 6 minutes à la température du laboratoire, après quoi, on a arrêté la réaction par addition d'hydroxyde de sodium normal, goutte à goutte, jusqu'à pH 7,5 (± 0,5). L'ajustement du pH était suivi d'un changement de coloration de l'indicateur rouge de phénol présent dans la suspension virale.

La suspension de virus traité a été immédiatement dialysée pendant 5 heures à 5° (± 1) dans un tampon phosphate consistant en NaCl (8 g); KCl (0,2 g); Na$_2$HPO$_4$ (1,15 g): KH$_2$PO$_4$ (0,2 g) dans de l'eau distillée (jusqu'à 800 ml) mélangé à une solution de MgCl$_2$ · 6H$_2$O dans 100 ml d'eau distillée et ensuit à une solution de CaCl$_2$ (0,1 g) dans 100 ml d'eau distillée, la solution finale étant stérilisée par filtration, le pH final étant compris entre 7,2 et 7,4). Après dialyse, la suspension de virus a été stockée dans de l'azote liquide.

Les tests de titration d'infectivité ont été effectués à 37°C en utilisant 4 tubes par dilution décimale. L'infectivité résiduelle de la suspension virale obtenue correspondait à $10^{1,7}$ DICT$_{50}$/0,1 ml de l'infectivité de la suspension de la souche virale C.N.C.M. I - 198 de départ. Des aliquotes (0,1 ml) de suspension virale diluées 1/9 dans un mélange 50/50 (v/v) de milieu de base et minimal de Eagle additionné de 0,25 % (poids/volume) d'hydrolysat de lactalbumine ont été inoculés à 80 cultures de la ligne cellulaire de rein de boeuf NLBK$_4$ qui ont ensuite été incubées à 37°C pendant 5 jours. Parmi les 80 tubes, 31 ont montré un effet cytopathogène dû à la multiplication des virus.

Chacune de ces 31 cultures a été cultivée et titrée comparativement à 35°C et à 39,5°C. Ces températures d'incubation ont été choisies respectivement comme température permissive et restrictive parce que le virus de départ (C.N.C.M. I - 198) se développait également bien à ces deux températures mais que sa culture était limitée à 40°. Un des isolats a montré une différence en titre de 3,0 log$_{10}$ DICT$_{50}$ (dose infectieuse en culture de tissu dans 50 % des cas entre 35° et 39,5°C, démontrant ainsi son caractère thermosensible. On l'a enrichi par un passage supplémentaire (le 19ème) dans les mêmes conditions opératoires que pour les passages précédents et on l'a ensuite lyophilisé.

On a multiplié le virus par deux passages sur une lignée cellulaire de cornets de boeuf (ici repris sous la dénomination NLBT), en utilisant le même milieu que celui qui est indiqué ci-avant et on a préparé un stock de virus au niveau de passage 21.

A partir de ce stock de virus, on a effectué un autre passage (clonage) à une dilution $10^{-4,5}$ dans les mêmes conditions en utilisant 96 cultures parmi lesquelles 8 étaient positives. L'une d'elles a été enrichie par un passage supplémentaire (24ème) dans les mêmes conditions pour donner un lot de semence qui a été lyophilisé dans des récipients en verre contenant chacun $10^{3,9}$ DICT$_{50}$ de virus. Des échantillons du clone ont été déposés le 10 juillet 1982 en accord avec les dispositions de la Convention sur le Brevet Européen dans

4

la Collection Nationale de Cultures de Microorganismes (C.N.C.M.) de l'Institut Pasteur à Paris, sous le numéro d'ordre I - 199.

## Exemple 2

### Préparation de vaccin

Un échantillon du lot d'ensemencement (C.N.C.M I - 199) a été réhydraté et inoculé sur des cultures de la lignée cellulaire NLBT, qui ont alors été recouvertes de milieu de maintenance, comme décrit ci-avant.

Les cellules ont été incubées pendant 5 jours à température permissive (35° ± 0,5). Le surnageant a ensuite été fraîchement inoculé à une autre série de cultures. Lorsqu'on a constaté l'apparition d'un effet cytopathogène sur 50 % du tapis cellulaire, les surnageants furent récoltés. On les a ensuite mélangés, on y a ajouté de la casitone (6 %, poids/volume) et stocké le mélange à - 70°C. Cette suspension virale a été décongelée, répartie dans des flacons en verre et lyophilisée, chaque flacon contenant $10^4$ DICT50 de virus ou un multiple de $10^4$ DICT50.

Avant administration, le vaccin est réhydraté extemporanément avec du sérum physiologique (NaCl 0,9 % dans de l'eau distillée stérile) en utilisant 2 ml par dose. Le vaccin est administré par voie intramusculaire et, de préférence, on administre deux doses, par exemple à un intervalle de 5 semaines.

## Exemple 3

### Thermosensibilité de la souche modifiée

La propriété de former des plages (PFP) exprimée en $\log_{10}$ UFP (unités formant plages) par 0,2 ml après incubation à différentes températures a été déterminée pour chacune des souches C.N.C.M. I - 198 et I - 199. Les résultats sont indiqués dans le Tableau 1 qui montre que PFP est compris entre $10^{4,5}$ et $10^{5,0}$ pour la souche sauvage C.N.C.M. I - 198 alors que le mutant C.N.C.M. I - 199 a une PFP optimale à 35 - 37°C qui est réduite de 1,5 $\log_{10}$ à 39°C et de 3,0 $\log_{10}$ à 39,5°C alors qu'on ne recueille pas de virus infectieux à partir des cultures inoculées qui ne présentent pas un effet cytopathogène à 39,5° C.

### Tableau 1

| Souche (PFP) à | Propriété de former des plages | | | | |
|---|---|---|---|---|---|
| | 35°C | 37°C | 38,5°c | 39°C | 39,5°C |
| CNCM I - 198 | 4,8 | 4,8 | NT | NT | 4,8 |
| CNCM I - 199 | 4,2 | 4,2 | 3,6 | 2,7 | 1,2 |

On a également déterminé les titres à 35°C et à 39,5°C pour le virus à différents niveaux de passages et les résultats sont donnés dans le Tableau 2; ils montrent que la différence entre les titres à 35°C et les titres à 39,5°C est stable au cours des différents passages.

### Tableau 2

| virus | Titres comparatifs sur lignée cellulaire NLBT, à température permissive et restrictive | | | |
|---|---|---|---|---|
| | niveau de passage | nombre de tests | rapport 35°/39°C (*) moyenne géométrique | déviation standard |
| CNCM I - 198 | 17 | 17 | 0,2 | 0,1 |
| CNCM I - 199 | 21 | 6 | 2,4 | 0,3 |
| | 23 | 2 | 2,1 | |
| | 24 | 15 | 3,0 | 0,4 |
| | 28 | 16 | 3,2 | 0,4 |

(*) log DITC10 ou UFP à 35°C
log DITC50 ou UFP à 39,5°C

## Exemple 4

### Atténuation pour les veaux normaux.

Le lot d'ensemencement de vaccin a été testé chez cinq animaux séronégatifs. Dans ce but, on a réhydraté 10 doses de vaccin dans 2 ml de sérum physiologique et on les a inoculées à chaque animal par voie intramusculaire. Trois semaines plus tard, on a, de la même manière, administré une seconde dose à chacun des 5 animaux.

Chaque jour, pendant les 14 jours qui ont suivi l'administration de la première dose, on a journellement effectué un examen clinique des animaux, on a enregistré leur température rectale et on a effectué un comptage de leurs globules blancs.

On n'a observé aucun symptôme. Comme le montre le Tableau 3, on n'a noté aucun élévation de température supérieure à 39,2°C.

### Tableau 3

| Jour M | Températures rectales enregistrées (en °C) | | | | |
| | | | veau n° | | |
| (X) | 010 | 012 | 431 | 432 | 433 |
|---|---|---|---|---|---|
| - 10 | 38,2 | 38,5 | 38,5 | 38,7 | 38,6 |
| - 9 | 38,0 | 38,0 | 38,5 | 38,7 | 38,6 |
| - 8 | 38,1 | 38,8 | 38,8 | 38,7 | 39,2 |
| - 7 | 38,0 | 38,0 | 39,0 | 38,8 | 38,7 |
| - 3 | 38,2 | 28,0 | 38,8 | 39,0 | 38,7 |
| - 2 | 38,0 | 38,2 | 39,0 | 38,9 | 38,7 |
| - 1 | 38,5 | 38,2 | 38,2 | 38,6 | 38,0 |
| 0 | 38,3 | 38,0 | 39,0 | 38,9 | 38,7 |
| 1 | 38,4 | 38,0 | 38,7 | 38,9 | 38,8 |
| 5 | 38,3 | 38,3 | 38,9 | 38,6 | 38,6 |
| 6 | 38,0 | 38,2 | 38,8 | 38,6 | 38,6 |
| 7 | 38,2 | 38,2 | 38,5 | 38,8 | 38,4 |
| 8 | 38,4 | 38,6 | 39,0 | 38,7 | 38,5 |
| 11 | 38,2 | 38,4 | 38,8 | 38,5 | 38,5 |
| 12 | 38,4 | 38,4 | 38,8 | 38,5 | 38,0 |
| 13 | 38,4 | 38,2 | 38,4 | 38,4 | 38,4 |
| 14 | 38,6 | 38,1 | 38,8 | 39,0 | 28,7 |

(X) le jour 0 est celui de l'inoculation de la première dose.

Le nombre moyen de globules blancs (NMGB) est resté normal pendant toute la période d'observation.

Comme le montre la séroconversion des animaux contre le VDB, ils ont tous été immunisés.

## Exemple 5

### Atténuation pour les animaux immunodéprimés

Neuf veaux séronégatifs au VDB et au virus respiratoire syncytial bovin (VRSB) ont été traités journellement avec 0,1 mg/kg de dexaméthasone pendant une période de 12 jours consécutifs commençant 5 jours avant l'administration de la première dose de vaccin.

Quatre animaux (Nos 16, 30, 82 et 82) ont reçu une inoculation intramusculaire du lot d'ensemencement le jour 0 et ils ont reçu par la même voie une dose identique au jour 21. La vaccination au jour 0 est reprise ici sous la dénomination 'primo-vaccination', la dose de vaccin utilisée pour cette première vaccination étant de $10^5$ $DICT_{50}$ de virus obtenu dans l'exemple 2.

Aux jours 0 et 21, trois animaux (à savoir: Nos 13, 14 et 19) ont été vaccinés avec un vaccin combiné VDB/VRSB contenant $10^{5,7}$ $DICT_{50}$ de VRSB et $10^5$ $DICT_{50}$ de VDB. Deux animaux (à savoir les nos 80 et 81) ont été vaccinés aux jours 0 et 21 avec le même vaccin VRSB monovalent contenant $10^{5,7}$ $DICT_{50}$ de virus par dose.

Pendant les 14 jours qui ont suivi l'administration de la première dose, on a effectué journellement un examen clinique des animaux, on a enregistré leur température interne et on a effectué un comptage de leurs globules blancs.

Chaque jour, on a également essayé d'isoler du virus d'échantillons de sang, d'écouvillonnages oculonasaux et de matières fécales de quatre animaux inoculés pendant la même période. Les résultats et leur interprétation sont donnés dans les Tableaux 4 et 5.

**Tableau 4**

**Scores Cliniques**

| Veau N° | 0 | 1 | 2 | 3 | 4 | 5 | 6 | 7 | 8 | 9 | 10 | 11 | 12 | 13 | 14 | 15/21 | Moyenne Individ. | Groupe |
|---|---|---|---|---|---|---|---|---|---|---|---|---|---|---|---|---|---|---|
| | | | | | | Scores Journaliers (jours après la première vaccination) | | | | | | | | | | | Moyenne | |
| 30 | - | - | - | - | - | - | 80 | 70 | 70 | 30 | -- | -- | -- | -- | --- | -- | 11,9 | |
| 83 | - | - | - | 30 | 30 | 30 | 50 | 50 | 40 | 40 | 40 | 10 | 10 | 10 | 10 | -- | 16,7 | 15,25 |
| 82 | - | - | - | - | - | - | 50 | 50 | 50 | 60 | 50 | 50 | 50 | 50 | 20 | -- | 20,5 | |
| 16 | - | - | 20 | 20 | 20 | 20 | 30 | 30 | 30 | 30 | 20 | 10 | 10 | 30 | 10 | -- | 11,9 | |
| Moyenne Journaliere | | | 5 | 12,5 | 12,5 | 12,5 | 52,5 | 50,0 | 47,5 | 40,0 | 27,5 | 17,5 | 17,5 | 22,5 | 10,0 | -- | | |
| 19 | - | - | - | - | - | - | 20 | -- | -- | 30 | -- | -- | -- | ---- | | -- | 2,4 | |
| 13 | - | - | - | 30 | 30 | 30 | 60 | 60 | 60 | 60 | 60 | 10 | 10 | -- | -- | -- | 19,5 | 15,20 |
| 14 | - | - | 30 | 30 | 30 | 30 | 50 | 50 | 80 | 60 | 60 | 30 | 30 | 10 | 10 | -- | 23,8 | |
| Moyenne Journaliere | | | 7,5 | 15,0 | 15,0 | 15,0 | 40,0 | 35,0 | 35,0 | 37,5 | 30,0 | 10,0 | 10,0 | 2,5 | 2,5 | -- | | |
| 80 | - | - | - | - | - | - | . | . | . | . | -- | -- | -- | -- | -- | -- | 0 | |
| 81 | - | - | - | - | - | - | 30 | 30 | 30 | . | -- | -- | -- | -- | -- | -- | 4,3 | 2,15 |
| Moyenne Journaliere | | | | | | | 15,0 | 15,0 | 15,0 | | | | | | | | | |

- : négatif

**Tableau 5**

**Methode de calcul des scores cliniques.**

| Paramètre | Degré | Score Unité (1) | Score Pondéré (2) | Score Clinique |
|---|---|---|---|---|
| Apathie | aucune | 0 | | Somme de |
| | léger | 1 | 10 | |
| | décubitus | 2 | | |
| Anorexie | aucune | 0 | | (1) |
| | appétit réduit | 1 | 20 | |
| | complète | 2 | | |
| Diarrhée | fèces normales | 0 | | Multiplié |
| | fèces molles | 1 | | |
| | fèces aqueuses | 2 | 10 | |
| | melaena | 3 | | |
| | nécrose | 4 | | par (2) |

Il apparaît qu'on observe différents degrés d'anorexie et de diarrhée chez tous les animaux vaccinés. Toutefois, aucun d'eux ne manifeste soit une hyperthermie, soit une leucopénie et on n'a pas isolé de VDB des échantillons recueillis. On n'a observé ni pathogénicité cumulée ni interférence résultant de la combinaison VRSB et VDB.

**Exemple 6**

**Efficacité de la souche C.N.C.M. I - 199 contre un challenge artificiel.**

Les animaux vaccinés pendant le traitement immunodépressif ont séroconverti après administration de la seconde dose. Au jour 35 après administration de la première dose, on leur a administré une inoculation d'épreuve ainsi qu'aux deux témoins.

Le VDB utilisé pour le challenge était la souche Osloss (RHODE G. et LIESS B., Zbl. Vet. Med. 17B : 686, 1970). Il a été administré par voie intranasale en utilisant $10^{6,8}$ DICT$_{50}$ par animal.

Pendant les 14 jours qui ont suivi le challenge, on a observé les symptômes cliniques, on a enregistré les températures corporelles et on a effectué un comptage des globules blancs. On a essayé de réisoler du virus du sang de tous les animaux. On n'a observé aucun symptôme clinique, ni hyperthermie.

Les veaux qui avaient reçu deux doses du vaccin ne présentaient aucune diminution du nombre de leurs globules blancs après challenge tandis que les témoins (animaux Nos 80 et 81) étaient leucopéniques. Même à la dose élevée de $10^{6,8}$ DICT$_{50}$/animal, la souche Osloss n'était pas pathogène. Chez les animaux N° 80 et 81 qui avaient été vaccinés avec le vaccin viral VRSB seulement, on n'a observé ni hyperthermie ni symptômes cliniques. Toutefois, ces deux animaux présentent un nombre de globules blancs inférieur à celui des veaux qui avaient reçu deux doses de vaccin VDB et une virémie a été mise en évidence chez ces animaux. Les animaux vaccinés avec le vaccin VDB/VRSB combiné étaient également bien protégés (Nos 19, 13 et 14).

Les résultats sont résumés dans le Tableau 6.

**Tableau 6**

| Veau N° | Vaccin | Virémie après challenge Jour après inoculation | | | | | | | | | | Index |
|---|---|---|---|---|---|---|---|---|---|---|---|---|
| | | 0 | 2 | 4 | 5 | 6 | 7 | 8 | 9 | 10 | 13 | |
| 16 | VDB | - | - | - | - | - | - | - | - | - | - | |
| 30 | | - | - | - | - | - | - | - | - | - | - | |
| 82 | | - | - | - | - | - | + | - | + | - | - | |
| 83 | | - | - | - | - | - | - | - | - | - | - | |
| | | | | | | | | | | | | 2/68 |
| 13 | VDB/VRSB | - | - | - | - | - | NT | - | - | - | - | |
| 14 | | - | - | - | - | - | NT | - | - | - | - | |
| 19 | | - | - | - | - | - | - | - | - | - | - | |
| 80 | VRSB | - | - | + | + | + | + | + | - | - | - | |
| 81 | | - | - | - | + | + | + | + | - | - | - | 9/20 |

- IF (test d'immunofluorescence) négatif
+ IF (test d'immunofluorescence) positif
NT: non testé

Ces résultats ne sont pas en opposition avec ceux rapportés dans la littérature puisque l'on sait que l'infection expérimentale par un phénotype sauvage VDB aboutit rarement à des symptômes cliniques graves (SAURAT P. et al., La Maladie des Muqueuses) Ed. L'Expansion Scientifique Française, Paris, 1972, p. 76).

L'index (c'est-à-dire le nombre d'échantillons positifs par rapport au nombre d'échantillons testés) était significativement plus élevé (9/20) pour le groupe d'animaux vaccinés avec le vaccin VRSB seul que pour les autres veaux parmi lesquels deux veaux étaient transitoirement positifs.

**Exemple 7**

**Efficacité de la souche C.N.C.M. I - 199 sur le terrain.**

On a administré deux doses de $10^4$ DICT$_{50}$ du vaccin de l'exemple 2 par voie intramusculaire, à trois semaines d'intervalle à cent et trois veaux âgés de 15 jours à 18 mois et présentant des titres d'anticorps faibles (c-à-d ≤ 8) à l'égard du VDB. On n'a constaté aucune réaction clinique imputable au vaccin, ce qui démontre la sécurité d'emploi de ce vaccin dans les conditions de la pratique.

Les résultats sont résumés dans le Tableau 7.

**Tableau 7**

| Titre d'anticorps avant vaccination | Nombre de séroconvertis/Nombre total d'animaux | | | | | | |
|---|---|---|---|---|---|---|---|
| | ≤ 1 | | | 1 - 8 | | | Total |
| Age (+) | ≤ 2 | > 2 | Total | ≤ 2 | > 2 | Total | Total |
| Nombres totaux | 9/20 | 51/57 | 60/77 | 2/6 | 6/10 | 8/16 | 68/90 |
| Pourcentages | 45 | 90 | 78 | 30 | 60 | 50 | 73 |

Comme il ressort du Tableau 7, 73 % des animaux ont présenté un accroissement significatif en anticorps. Toutefois, en considérant seulement les veaux qui étaient séronégatifs à la vaccination, il y avait une nette différence (p < 0.001) du taux de séroconversion des animaux âgés de plus de deux mois: leur séroconversion était de 90 % alors que le taux de séroconversion observé chez les animaux de moins de 2 mois était de 45 %. Les animaux séropositifs ont manifesté la même tendance. On a observé une meilleure réponse chez les animaux plus âgés.

Chez les animaux séonégatifs âgés de plus de deux mois, la souche thermosensible C.N.C.M. I - 199 a induit 90 % de séroconversion après deux doses de $10^4$ DICT$_{50}$. Cette observation ne contraste pas avec le taux de 95 à 98 % obtenu dans les conditions optimales avec les vaccins commercialement disponibles (LAMBERT G. et al., Modern. Vet. Practice p. 34 Avril 1970). Le taux de séroconversion de 50 % observé chez les veaux âgés de moins de deux mois est en accord avec les résultats obtenus par SMITH P.E. et al., (Vet. Med. Small. Anim. 63 : 457; 1968) qui ont signalé une réponse positive à la vaccination chez 5/10 des animaux âgés de moins de 60 jours et chez 42/48 des animaux plus âgés qui, à la vaccination, présentaient un statut sérologique analogue. Dans le présent essai, seize animaux séropositifs ont été vaccinés et 50 % ont séroconverti. Bien que ces nombres soient petits, ces résultats indiquent que l'on peut immuniser des animaux âgés de plus de deux mois qui présentent des titres d'anticorps faibles à la vaccination.

**Revendications** pour les Etats Contractants: BE, CH, DE, FR, GB, IT, LI, LU, NL, SE.

1. Vaccin vivant contre le virus de la diarrhée virale bovine capable d'induire une immunité chez les bovins, *caractérisé* en ce qu'il comprend une quantité efficace d'un mutant thermosensible (ts) du virus VDB.

2. Vaccin suivant la revendication 1, dans lequel le mutant ts montre une différence en titre infectieux d'environ 3 $\log_{10}$ DICT$_{50}$ entre 35°C et 39,5°C.

3. Vaccin suivant la revendication 1, dans lequel la quantité efficace est au moins $10^{3,5}$ DICT$_{50}$ de mutant ts.

4. Vaccin suivant la revendication 1, 2 ou 3, dans lequelle mutant ts est obtenu par mutagénèse avec un agent chimique mutagène.

5. Vaccin suivant la revendication 4, dans lequel l'agent chimique mutagène est l'acide nitreux.

6. Vaccin suivant la revendication 5, *caractérisé* en ce qu'on effectue la mutagénèse en mettent la souche virale VDB en contact avec l'acide nitreux pendant 1 à 15 minutes dans un milieu aqueux tamponné à un pH compris entre 5 et 6.

7. Vaccin suivant la revendication 1, 2 ou 3, lorsqu'il est combiné avec au moins un autre vaccin vivant à base de virus respiratoire bovin.

8. Vaccin suivant la revendication 1, 2 ou 3, lorsqu'il est combiné à un vaccin vivant à base de virus respiratoire syncytial bovin (VRSB).

9. Vaccin suivant la revendication 1, 2 ou 3, dans lequel le mutant ts est la souche C.N.C.M. I - 199 du virus VDB.

10. Souche virale C.N.C.M. I - 199.

11. Procédé de préparation d'une souche modifiée du virus de la diarrhée virale bovine utile pour un usage vaccinal, *caractérisé* en ce qu'on met une souche de virus de la diarrhée virale bovine en contact à température ordinaire avec un agent chimique mutagène dans des conditions opératoires qui réduisent le titre initial en virus de 2 à 3 $\log_{10}$ et que l'on isole par culture et titration, respectivement à 35°C et 39,5°C, un mutant thermosensible montrant une différence de titre d'environ 3 $\log_{10}$ DICT$_{50}$ entre 35° et 39,5°C.

12. Procédé suivant la revendication 11, dans lequel l'agent chimique mutagène est l'acide nitreux.

13. Procédé suivant la revendication 12, dans lequel la souche de virus est mise en contact avec l'acide nitreux pendant 1 à 15 minutes dans un milieu aqueux tamponné à un pH compris entre 5 et 6.

14. Procédé suivant la revendication 13, dans lequel la période de réaction est de 6 minutes (± 1), le pH est égal à 5,7 (± 0,1) et le milieu réactionnel est l'acide nitreux 4N dans un tampon acide acétique/ acétate normal.

15. Procédé suivant la revendication 14, dans lequel la souche modifiée isolée est la souche C.N.C.M. I - 199.

16. Procédé de préparation d'un vaccin contre le virus de la diarrhée virale bovine (VDB) qui consiste à cultiver le mutant isolé, suivant le procédé de la revendication 11, dans une culture cellulaire appropriée de façon à permettre la multiplication dudit mutant et à combiner le virus modifié avec un excipient en vue de l'administration parentérale.

17. Procédé suivant la revendication 16, dans lequel le mutant isolé est la souche C.N.C.M. I - 199.

18. Procédé suivant les revendications 16 ou 17, dans lequel le virus modifié est, en outre, mélangé à un autre vaccin vivant à base de virus respiratoire bovin.

19. Procédé suivant la revendication 18, dans lequel l'autre vaccin vivant à base de virus respiratoire bovin est un vaccin vivant à base de virus respiratoire syncytial bovin (VRSB).

**Revendication** pour l'Etat Contractant AT.

1. Procédé de préparation d'une souche modifiée du virus de la diarrhée virale bovine utile pour un usage vaccinal, *caractérisé* en ce qu'on met une souche de virus de la diarrhée virale bovine en contact à température ordinaire avec un agent chimique mutagène dans des conditions opératoires qui réduisent le titre initial en virus de 2 à 3 $\log_{10}$ et que l'on isole par culture et titration, respectivement à 35°C et 39,5°C, un mutant thermosensible montrant une différence de titre d'environ 3 $\log_{10}$ DICT$_{50}$ entre 35° et 39,5°C.

2. Procédé suivant la revendication 1, dans lequel l'agent chimique mutagène est l'acide nitreux.

3. Procédé suivant la revendication 2, dans lequel la souche de virus est mise en contact avec l'acide nitreux pendant 1 à 15 minutes dans un milieu aqueux tamponné à un pH compris entre 5 et 6.

4. Procédé suivant la revendication 3, dans lequel la période de réaction est de 6 minutes (± 1), le pH est égal à 5,7 (± 0,1) et le milieu réactionnel est l'acide nitreux 4N dans un tampon acide acétique/ acétate normal.

5. Procédé suivant la revendication 4, dans lequel la souche modifiée isolée est la souche C.N.C.M. I - 199.

6. Procédé de préparation d'un vaccin contre le virus de la diarrhée virale bovine (VDB) qui consiste à cultiver le mutant isolé, suivant le procédé de la revendication 1, dans une culture cellulaire appropriée de façon à permettre la multiplication dudit mutant et à combiner le virus modifié avec un excipient en vue de l'administration parentérale.

7. Procédé suivant la revendication 6, dans lequel le mutant isolé est la souche C.N.C.M. I - 199.

8. Procédé suivant les revendications 6 ou 7, dans lequel le virus modifié est, en outre, mélangé à un autre vaccin vivant à base de virus respiratoire bovin.

9. Procédé suivant la revendication 9, dans lequel l'autre vaccin vivant à base de virus respiratoire bovin est un vaccin vivant à base de virus respiratoire syncytial bovin (VRSB).

**Patentansprüche** (für die Vertragsstaaten: BE, CH, DE, FR, GB, IT, LI, LU, NL, SE)

1. Lebendimpfstoff gegen das Rinderdiarrhoe-Virus, der in der Lage ist, bei Rindern Immunität zu induzieren, dadurch gekennzeichnet, daß er eine wirksame Menge einer temperatursensitiven Mutante (ts) des RDV-Virus enthält.

2. Impfstoff nach Anspruch 1, bei dem die ts-Mutante bei einer Temperatur zwischen 35°C und 39,5°C eine Differenz im infektiösen Titer von etwa 3 $\log_{10}$ DICT$_{50}$ aufweist.

3. Impfstoff nach Anspruch 1, bei dem die wirksame Menge mindestens $10^{3,5}$ DICT$_{50}$ der ts-Mutante beträgt.

4. Impfstoff nach einem der Ansprüche 1, 2 oder 3, bei dem die ts-Mutante durch Mutagenese mit einem chemischen Mutagen erhalten wird.

5. Impfstoff nach Anspruch 4, bei dem das chemische Mutagen Salpetersäure ist.

6. Impfstoff nach Anspruch 5, *dadurch gekennzeichnet*, daß man zur Durchführung der Mutagenese den RDV-Virus-Stamm 1 bis 15 Minuten mit der Salpetersäure in einer wäßrigen Pufferlösung mit einem pH-Wert zwischen 5 und 6 inkubiert.

7. Impfstoff nach einem der Ansprüche 1, 2 oder 3, mit der Maßgabe, daß er mit mindestens einem anderen Lebendimpfstoff aus dem Rinder-Atemwege-Virus kombiniert wird.

8. Impfstoff nach einem der Ansprüche 1, 2 oder 3, mit der Maßgabe, daß er mit einem Lebendimpfstoff aus dem Rinder-Atemwege-Synzytien Virus (RASV) kombiniert wird.

9. Impfstoff nach einem der Ansprüche 1, 2 oder 3, bei dem die ts-Mutante der Stamm C.N.C.M I - 199 des RDV-Virus ist.

10. Virus-Stamm C.N.C.M. I - 199.

11. Verfahren zur Herstellung eines für Impfzwecke geeigneten modifizierten Stammes des Rinderdiarrhoe-Virus, *dadurch gekennzeichnet*, daß man einen Virus-Stamm der viralen Rinderdiarrhoe bei Normaltemperatur mit einem chemischen Mutagen unter Verfahrensbedingungen inkubiert, bei denen sich der Ausgangstiter des Virus um 2 bis 3 $\log_{10}$ reduziert, und daß man eine temperatursensitive Mutante, die bei einer Temperatur zwischen 35°C und 39,5°C eine Titerdifferenz von etwa 3 $\log_{10}$ DICT$_{50}$ aufweist, durch Züchtung und Titration bei 35°C bzw. 39,5°C isoliert.

12. Verfahren nach Anspruch 11, bei dem das chemische Mutagen Salpetersäure ist.

13. Verfahren nach Anspruch 12, bei dem der Virus-Stamm mit der Salpetersäure 1 bis 15 Minuten in einer wäßrigen Pufferlösung mit einem pH-Wert zwischen 5 und 6 inkubiert wird.

14. Verfahren nach Anspruch 13, bei dem die Reaktionszeit 6 (± 1) Minuten beträgt, der pH-Wert 5,7 (± 0,1) ist und das Reaktionsmedium 4N Salpetersäure in einem üblichen Essigsäure/Acetat-Puffer ist.

15. Verfahren nach Anspruch 14, bei dem der isolierte modifizierte Stamm der Stamm C.N.C.M. I - 199 ist.

16. Verfahren zur Herstellung eines Impfstoffes gegen das Virus der viralen Rinderdiarrhoe (RDV), bei dem man die nach dem Verfahren von Anspruch 11 isolierte Mutante in einer geeigneten Zellkultur züchtet, die die Vermehrung der Mutante sowie die Kombination des modifizierten Virus mit einem Exzipienz für die parenterale Verabreichung gestattet.

17. Verfahren nach Anspruch 16, bei dem die isolierte Mutante der Stamm C.N.C.M. I - 199 ist.

18. Verfahren nach einem der Ansprüche 16 oder 17, bei dem das modifizierte Virus außerdem mit einem anderen Lebendimpfstoff aus dem Rinder-Atemwege-Virus versetzt wird.

19. Verfahren nach Anspruch 18, bei dem der andere Lebendimpfstoff aus dem Rinder-Atemwege-Virus ein Lebendimpfstoff aus dem Rinder-Atemwege-Synzytien-Virus (RASV) ist.

**Patentansprüche** (für den Vertragsstaat AT)

1. Verfahren zur Herstellung eines für Impfzwecke geeigneten modifizierten Stammes des Rinderdiarrhoe-Virus, *dadurch gekennzeichnet*, daß man einen Virus-Stamm der viralen Rinderdiarrhoe bei Normaltemperatur mit einem chemischen Mutagen unter Verfahrensbedingungen inkubiert, bei denen sich der Ausgangstiter des Virus um 2 bis 3 $\log_{10}$ reduziert und daß man eine temperatursensitive Mutante, die bei einer Temperatur zwischen 35°C und 39,5°C eine Titerdifferenz von etwa 3 $\log_{10}$ DICT$_{50}$ aufweist, durch Züchtung und Titration bei 35°C, bzw. 39,5°C, isoliert.

2. Verfahren nach Anspruch 1, bei dem das chemische Mutagen Salpetersäure ist.

3. Verfahren nach Anspruch 2, bei dem der Virus-Stamm mit der Salpetersäure 1 bis 15 Minuten in einer wäßrigen Pufferlösung mit einem pH Wert zwischen 5 und 6 inkubiert wird.

4. Verfahren nach Anspruch 3, bei dem die Reaktionszeit 6 Minuten (± 1) beträgt, der pH Wert 5,7 (± 0,1) ist und das Reaktionsmedium 4N Salpetersäure in einem üblichen Essigsäure/Acetat-Puffer ist.

5. Verfahren nach Anspruch 4, bei dem der isolierte modifizierte Stamm der Stamm C.N.C.M. I - 199 ist.

6. Verfahren zur Herstellung eines Impfstoffes gegen das Virus der viralen Rinderdiarrhoe (RDV), bei dem man die isolierte Mutante nach dem Verfahren von Anspruch 1 in einer geeigneten Zellkultur züchtet, die die Vermehrung der Mutante sowie die Kombination des modifizierten Virus mit einem Exzipienz für die parenterale Verabreichung gestattet.

7. Verfahren nach Anspruch 6, bei dem die isolierte Mutante der Stamm C.N.C.M. I - 199 ist.

8. Verfahren nach einem der Ansprüche 6 oder 7, bei dem das modifizierte Virus außerdem mit einem anderen Lebendimpfstoff aus dem Rinder-Atemwege-Virus versetzt wird.

9. Verfahren nach Anspruch 8, bei dem der andere Lebendimpfstoff aus dem Rinder-Atemwege-Virus ein Lebendimpfstoff aus dem Rinder-Atemwege-Synzytien-Virus (RASV) ist.

**Claims** for the contracting States: BE, CH, DE, FR, GB, IT, LI, LU, NL, SE

1. A live bovine viral diarrhoea virus vaccine capable of inducing immunity in bovines comprising an effective amount of a temperature sensitive (ts) mutant of BVD virus.

2. A vaccine according to claim 1 wherein the ts mutant shows a difference of infectious titer of about 3 $\log_{10}$ TCID$_{50}$ between 35°C and 39.5°C.

3. A vaccine according to claim 1 wherein the effective amount is at least $10^{3.5}$ TCID$_{50}$ of ts mutant.

4. A vaccine according to claims 1, 2 or 3 wherein the ts mutant is obtained by mutagenesis with a chemical mutagen.

5. A vaccine according to claim 4 wherein the chemical mutagen is nitrous acid.

6. A vaccine according to claim 5 wherein the mutagenesis is performed by bringing a BVD virus strain for one to 15 minutes into contact with nitrous acid in a buffered aqueous medium at a pH comprised between 5 and 6.

7. A vaccine according to claim 1, 2 or 3 when combined with at least one other live bovine respiratory virus vaccine.

8. A vaccine according to claim 1, 2 or 3 when combined with a live bovine respiratory syncytial (BRS) virus vaccine.

9. A vaccine according to claim 1, 2 or 3 wherein the ts mutant is the BVD virus strain C.N.C.M. I - 199.

10. The C.N.C.M. I - 199 virus strain.

11. A process for preparing a bovine viral diarrhoea (BVD) virus modified strain valuable for vaccinal use which comprises bringing a bovine viral diarrhoea virus strain into contact at room temperature with a chemical mutagen in operative conditions which reduce the initial virus titer by 2 to 3 $\log_{10}$ and isolating by cultivation and titration at 35°C and 39.5°C respectively a temperature sensitive mutant showing a difference of titer of about 3 $\log_{10}$ TCID$_{50}$ between 35 and 39.5°C.

12. A process according to claim 11 wherein the chemical mutagen is nitrous acid.

13. A process according to claim 12 wherein the BVD virus strain is brought into contact for one to 15 minutes with nitrous acid in a buffered aqueous medium at a pH comprised between 5 and 6.

14. A process according to claim 13 wherein the reaction period is 6 minutes (± 1), the pH is 5.7 (± 0.1) and the reaction medium is 4 N nitrous acid in normal acetic acid/acetate buffer.

15. A process according to claim 14 wherein the isolated modified strain is the strain C.N.C.M. I - 199.

16. A process for preparing a bovine viral diarrhoea (BVD) virus vaccine comprising growing in a suitable cell culture the mutant isolated by the process of claim 11 to permit growth of a greater amount of said mutant and combining the modified virus with a carrier for parenteral administration.

17. A process according to claim 16 wherein the isolated mutant is the C.N.C.M. I - 199 strain.

18. A process according to claims 16 or 17 wherein the modified virus is further mixed with another live bovine respiratory virus vaccine.

19. A process according to claim 18 wherein the other live bovine respiratory virus vaccine is a live bovine respiratory syncytial (BRS) virus vaccine.

Claims for the Contracting States: AT

1. Process for the preparation of a modified strain of bovine viral diarrhoea virus useful for vaccinal use, characterized in that a strain of bovine viral diarrhoea virus is brought into contact with a chemical mutagen at room temperature in such operative conditions that the initial virus titer is reduced from 2 to 3 $\log_{10}$ and there is isolated by culture and titration at 35°C and 39.5°C respectively a temperature-sensitive mutant showing a difference in titers of about 3 $\log_{10}$ TCID$_{50}$ between 35°C and 39.5°C.

2. Process according to claim 1 wherein the chemical mutagen is nitrous acid.

3. Process according to claim 2 wherein the virus strain is brought into contact with nitrous acid for 1 to 15 minutes in a buffered aqueous solution at a pH comprised between 5 and 6.

4. Process according to claim 3 wherein the reaction time is 6 minutes (± 1), the pH is 5.7 (± 1) and the reaction

EP 0 106 831 B1

medium is 4 N nitrous acid in N acetate/ acetic acid buffer.

5. Process according to claim 4 wherein the isolated modified strain is the strain C.N.C.M. I - 199.

6. Process for the preparation a vaccine against infection by bovine viral diarrhoea virus (BVD) which comprises growing a mutant isolated according to the process of claim 1 in an appropriate cell tissue culture in order to multiply said mutant and combining said modified virus with a carrier for parenteral administration.

7. Process according to claim 6 wherein the isolated mutant is the strain C.N.C.M. I - 199.

8. Process according to claims 6 or 7 wherein the modified virus is further mixed with another live bovine respiratory virus vaccine.

9. Process according to claim 18 wherein the other live bovine respiratory virus vaccine is a live bovine respiratory syncytial virus vaccine.

13